# EUROPEAN PATENT APPLICATION

(11) **EP 3 216 449 A1**
(43) Date of publication of application: **13.09.2017**
(21) Application number: 17166067.3
(22) Date of filing: 15.11.2004
(51) Int. Cl.: A61K 31/517, A61K 9/16, A61K 9/20, A61K 9/22, A61K 9/28, A61K 31/519, A61K 31/53, A61K 31/549, A61K 47/14, A61K 47/32, A61K 47/36

(54) **ORAL ADMINISTRATION PREPARATION OF PHENYLALANINE DERIVATIVES**

(30) Priority: 14.11.2003 JP 2003385502
(62) Divisional of application: 04818538.3
(71) Applicant: EA Pharma Co., Ltd., Tokyo 104-0042 (JP)
(72) Inventor: OGAWA, Kenichi, Kanagawa, 2108681 (JP); HAGIO, Hirokazu, Kanagawa, 2108681 (JP); HIGUCHI, Hiroyuki, Kanagawa, 2108681 (JP); ISHIKAWA, Motoki, Kanagawa, 2108681 (JP); YABUKI, Akira, Kanagawa, 2108681 (JP)
(74) Representative: Dunne, Paul David

(57) **Abstract**

The present invention provides oral administration preparations containing the phenylalanine compound of the formula (1) wherein A represent the formula (2) and the like; B represents an alkoxy group and the like; E represents a hydrogen atom and the like; D represents a substituted phenyl group and the like; T, U, and V represent a carbonyl group and the like; Arm represents a benzene ring and the like; R1 represents an alkyl group and the like; R2, R3, and R4 may be the same or different from each other and represent a hydrogen atom, substituted amino group and the like; and J and J' represent a hydrogen atom and the like, or pharmaceutically acceptable salts thereof as an active ingredient in either form of a coating preparation or a matrix preparation. This active ingredient has a relatively shorter half-life in blood plasma, and the preparations having continuance of the effects are provided by the present invention.

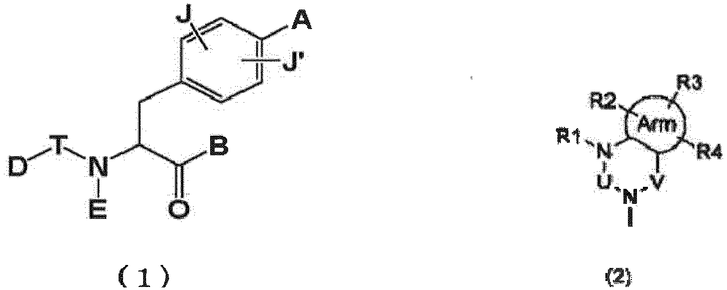

## Description

### Technical Field of the Invention

The present invention relates to sustained-release oral administration preparations of phenylalanine derivatives or pharmaceutically acceptable salts thereof, which have an α 4 integrin inhibiting activity and are useful as agents for treating inflammatory bowel disease and the like.

Especially it relates to coating preparations or matrix preparations.

### Background of the Invention

A compound of a formula (1) or pharmaceutically acceptable salts thereof have an α 4 integrin inhibiting activity and are useful compounds as agents for treating inflammatory bowel disease and the like. They can be produced in accordance with the description in Patent Literature 1. Though there is a description in this patent publication regarding ordinary tablets, granules, dispersants, pills, capsules, and sugarcoated agents, wherein the compound of the formula (1) or pharmaceutically acceptable salts thereof are dispensed, there is no disclosure on sustained-release oral administration preparations therein. Since the compound of the formula (1) or pharmaceutically acceptable salts thereof have a relatively shorter half-life period from blood plasma, QOL (quality of life) or compliance of patients may be improved by reducing doses of the above drugs.
[Patent Literature 1] WO02/16329

### Disclosure of the Invention

The object of the present invention is to provide a sustained-release oral administration preparation which allows the compound of the formula (1) or pharmaceutically acceptable salts thereof to exist longer in blood plasma.

The inventors have variously studied the above problem to solve it from the pharmaceutical point of view, and found that medicinal agents can become sustained-release by treating the compound of the formula (1) or pharmaceutically acceptable salts thereof with a water-soluble coating material(s) or a water-insoluble coating material(s) to prepare coating preparations; or with a water-soluble matrix material(s) to prepare matrix preparations. The present invention has been completed based on this finding.

Namely, the present invention relates to a sustained-release oral administration preparation containing a phenylalanine compound of the following formula (1) (hereinafter referred to as a compound (I)) or pharmaceutically acceptable salts thereof as an active ingredient: 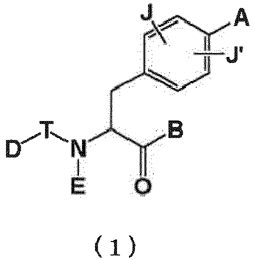 wherein A represents either of a following formula (2), (3), (3-1), or (3-2): 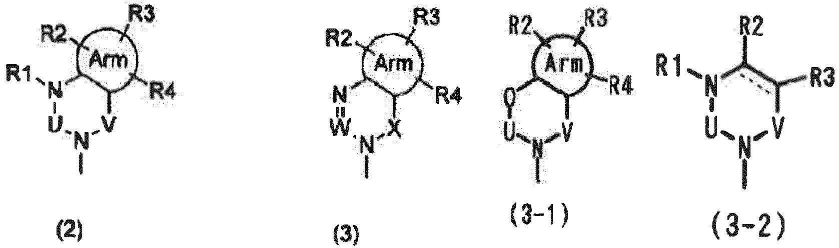 wherein Arm is a cyclic alkyl group or an aromatic ring having 0, 1, 2, 3, or 4 hetero atoms selected from the group consisting of an oxygen atom, sulfur atom and nitrogen atom; a combined line of a solid line and a dotted line in the formula (3-2) represents a single bond or a double bond; U, V, and X represent C(=O), S(=O)₂, C(-R5)(-R6), C(=C(R5)(R6)), C(=S), S(=O), P(=O)(-OH), or P(-H)(=O); W represents C(-R7) or a nitrogen atom;
wherein R1, R2, R3, R4, R5, R6 and R7 may be the same or different from each other and represent a hydrogen atom, halogen atom, hydroxyl group, lower alkyl group, substituted low alkyl group, lower alkenyl group, substituted lower alkenyl group, lower alkynyl group, substituted lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), lower alkyl group substituted with a heteroaryl group(s), lower alkoxy group, lower alkylthio group, lower alkoxy group and lower alkylthio group substituted with a cycloalkyl group(s) which may have a hetero atom(s) in the ring, lower alkoxy group and lower alkylthio group substituted with an aryl group(s), lower alkoxy group and lower alkylthio group substituted with a heteroaryl group(s), cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkylthio group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylsulfornyl group, substituted or unsubstituted sulfamoyl group, or ammonium group; R5 and R6 may bond together to form a ring, and in the ring, one or two oxygen atoms, nitrogen atoms or sulfur atoms may be included if necessary;
B represents a hydroxyl group, lower alkoxy group, or hydroxylamino group;
E represents a hydrogen atom, lower alkyl group, lower alkenyl group, lower alkynyl group, lower alkyl group substituted with a cycloalkyl group(s) which may have a hetero atom(s) in the ring, lower alkyl group substituted with an aryl group(s), or lower alkyl group substituted with a heteroaryl group(s);
D represents a lower alkyl group, lower alkenyl group, lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), or lower alkyl group substituted with a heteroaryl group(s), lower alkoxy group, lower alkoxy group substituted with a cycloalkyl group(s) which may have a hetero atom(s) in the ring, lower alkoxy group substituted with an aryl group(s), lower alkoxy group substituted with a heteroaryl group(s), cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylthio group, lower alkylsulfornyl group, or substituted or unsubstituted sulfamoyl group;
E and D may bond together to form a ring, and in the ring, one or two oxygen atoms, nitrogen atoms or sulfur atoms may be included if necessary;
T represents an interatomic bond, C(=O), C(=S), S(=O), S(=O)₂, N(H)-C(=O), or N(H)-C(=S); and
J and J' may be the same or different from each other and represents a hydrogen atom, halogen atom, lower alkyl group, lower alkyloxy group, or nitro group.

### Brief Description of the Drawings

Fig. 1 shows results of the dissolution test on Test Example 1. The horizontal axis indicates each example, and the vertical axis indicates the dissolution rate (wt%) of the compound (A) from a preparation two hours later.
Fig. 2 shows results of the dissolution test on Test Example 2. The horizontal axis indicates time (minutes), and the vertical axis indicates the dissolution rate (wt%) of the compound (A) from a preparation.
Fig. 3 shows results of the dissolution test on Test Example 2. The horizontal axis indicates time (minutes), and the vertical axis indicates the dissolution rate (wt%) of the compound (A) from a preparation.
Fig. 4 shows results of the dissolution test on Test Example 3. The horizontal axis indicates time (minutes), and the vertical axis indicates the dissolution rate (wt%) of the compound (A) from a preparation.

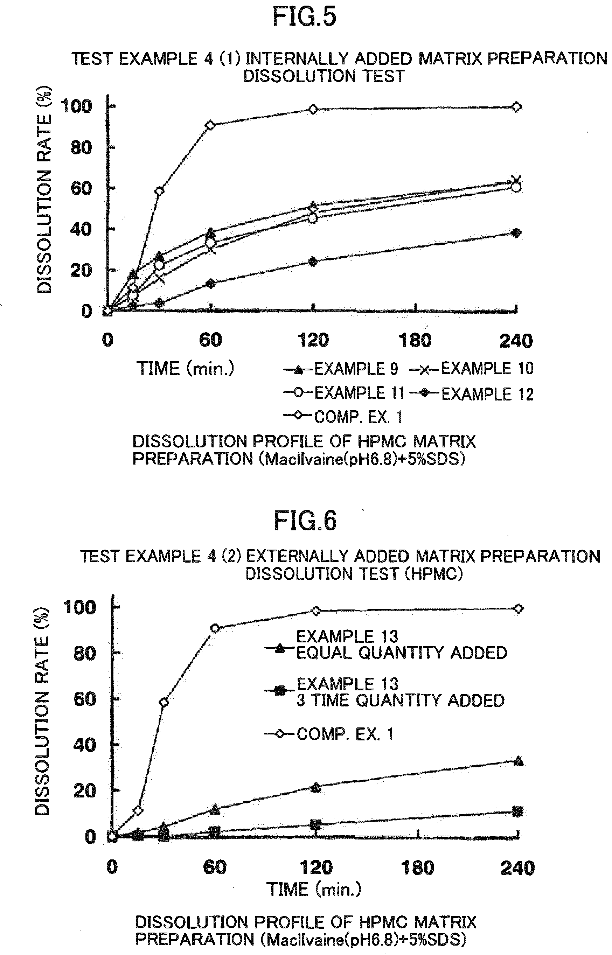

Fig. 5 shows results of the dissolution test on Test Example 4. The horizontal axis indicates each example, and the vertical axis indicates the dissolution rate (wt%) of the compound (A) from a preparation two hours later.
Fig. 6 shows results of the dissolution test on Test Example 4. The horizontal axis indicates time (minutes), and the vertical axis indicates the dissolution rate (wt%) of the compound (A) from a preparation.

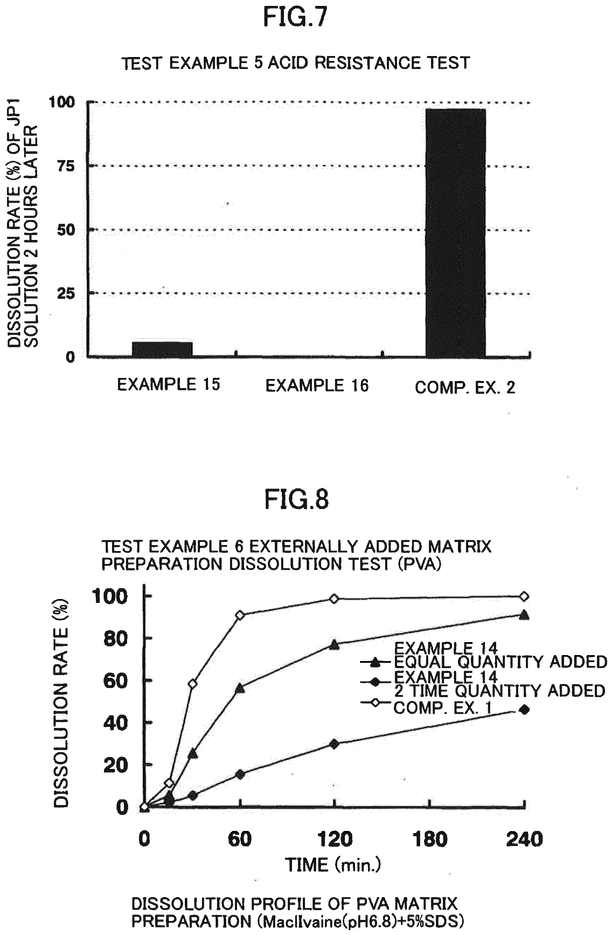

Fig. 7 shows results of the dissolution test on Test Example 5. The horizontal axis indicates time (minutes), and the vertical axis indicates the dissolution rate (wt%) of the compound (A) from a preparation.
Fig. 8 shows results of the dissolution test on Test Example 6. The horizontal axis indicates time (minutes), and the vertical axis indicates the dissolution rate (wt%) of the compound (A) from a preparation.

### Best Mode for Carrying out the Invention

In definitions of each group in the formulae (1), (2), (3-1), and (3-2) in the present specification, "lower" in a lower alkyl group and the like indicates a group having 1 to 6 carbon atoms and preferably having 1 to 4 carbon atoms. Alkyl groups, alkenyl group, and alkynyl groups as constituents of an alkyl group, alkenyl group, alkynyl group, alkoxy group, alkylthio group, alkanoyl group, alkylamino group, and the like can be linear or branched chains. The examples of alkyl groups include a methyl group, ethyl group, propyl group, isopropyl group, butyl group, sec-butyl group, tert-butyl group, pentyl group, and hexyl group, preferably having 1 to 6 carbon atoms and more preferably having 1 to 4 carbon atoms. The examples of alkenyl groups include a vinyl group, propenyl group, butenyl group, and pentenyl group, preferably having 2 to 6 carbon atoms and more preferably 2 to 4 carbon atoms. The examples of alkynyl groups include an ethynyl group, propynyl group, and butynyl group, preferably having 2 to 8 carbon atoms and more preferably 2 to 4 carbon atoms. A cycloalkyl group indicates a substituted or unsubstituted cycloalkyl group. The examples thereof include a cyclopropyl group, cyclobutyl group, cyclopentyl group, cyclohexyl group, norbornyl group, adamantyl group, and cyclohexenyl group, preferably having 3 to 8 carbon atoms and more preferably 3 to 5 carbon atoms. The examples of alkoxy groups include a methoxy group, ethoxy group, propyloxy group, and isopropyloxy group, preferably having 1 to 6 carbon atoms and more preferably 1 to 4 carbon atoms.

Hetero atoms include a nitrogen, oxygen, sulfur and the like. Halogen atoms represent a fluorine, chlorine, bromine and iodine. The examples of halogeno alkyl groups include a chloromethyl group, trichloromethyl group, trifluoromethyl group, trifluoroethyl group, and pentafluoromethyl group. The examples of halogeno alkoxy groups include a trichloromethoxy group and trifluoromethoxy group. The examples of hydroxyalkyl groups include a hydroxymethyl group and hydroxyethyl group. A cycloalkyl group which may have a hetero atom(s) in the ring may be substituted or unsubstituted, and the examples thereof include preferably 4- to 8-membered ring and more preferably 5- to 7-membered ring of a cyclopentyl group, cyclohexyl group, piperidyl group, piperazinyl group, morpholinyl group, pyrrolidinyl group, tetrahydrofuranyl group, and uracil group.

An aryl group indicates a substituted or unsubstituted aryl group and includes, for example, a phenyl group, 1-naphthyl group, and 2- naphthyl group. A phenyl group and a substituted phenyl group are preferable among them, and a halogen atom, alkoxy group, alkyl group, hydroxyl group, halogeno alkyl group, and halogeno alkoxy group are particularly preferable as a substituent(s). A heteroaryl group indicates a substituted or unsubstituted heteroaryl group and includes, for example, a pyridyl group, pyrazyl group, pyrimidyl group, pyrazolyl group, pyrrolyl group, triazyl group, furyl group, thienyl group, isoxazolyl group, isothiazolyl group, indolyl group, quinolyl group, isoquinolyl group, benzoimidazolyl group, and imidazolyl group. A pyridyl group, pyrazyl group, pyrimidyl group, furyl group, thienyl group, imidazolyl group and substituted pyridyl, furyl, thienyl groups are preferable among them. A halogen atom, alkoxy group, alkyl group, hydroxyl group, halogeno alkyl group, and halogeno alkoxy group are particularly preferable as a substituent(s). A lower alkyl group substituted with an aryl group(s) includes a substituted or unsubstituted benzyl group, and a substituted or unsubstituted phenethyl group, for example. A halogen atom, alkoxy group, alkyl group, hydroxyl group, halogeno alkyl group, and halogeno alkoxy group are particularly preferable as a substituent(s). A lower alkyl group substituted with a heteroaryl group(s) includes a pyridylmethyl group, for example. A halogen atom, alkoxy group, alkyl group, hydroxyl group, halogeno alkyl group, and halogeno alkoxy group are particularly preferable as a substituent(s).

Examples of alkanoyl groups include a formyl group, acetyl group, propanoyl group, butanoyl group, and pivaloyl group. Examples of aroyl groups include a substituted or unsubstituted benzoyl and pyridylcarbonyl groups. A halogen atom, alkoxy group, alkyl group, hydroxyl group, halogeno alkyl group, and halogeno alkoxy group are particularly preferable as a substituent(s). Examples of halogeno alkanoyl groups include a trichloroacetyl group and trifluoroacetyl group. Examples of alkylsulfonyl groups include a methanesulfonyl group and ethanesulfonyl group. Arylsulfonyl groups include a benzenesulfonyl group and p-toluenesulfonyl group. Heteroarylsulfonyl groups include a pyridylsulfonyl group. Halogeno alkylsulfonyl groups include a trifluoromethanesulfonyl group. Alkyloxycarbonyl groups include a methoxycarbonyl group, ethoxycarbonyl group, and tert-butoxycarbonyl group, and aryl-substituted alkoxycarbonyl groups include a benzyloxycarbonyl group and 9-fluorenylmethoxycarbonyl group.

Examples of substituted carbamoyl groups include a methylcarbamoyl group, phenylcarbamoyl group, and substituted phenylcarbamoyl group. A halogen atom, alkoxy group, alkyl group, hydroxyl group, halogeno alkyl group, and halogeno alkoxy group are particularly preferable as a substituent(s). Examples of substituted thiocarbamoyl groups include a methylthiocarbamoyl group, phenylthiocarbamoyl group, and substituted phenylthiocarbamoyl group. A halogen atom, alkoxy group, alkyl group, hydroxyl group, halogeno alkyl group, and halogeno alkoxy group are particularly preferable as a substituent(s). A substituted amino group in the present specification indicates a monosubstituted or disubstituted amino group. The substituents thereof include a lower alkyl group, lower alkyl group substituted with an aryl group(s), lower alkyl group substituted with a heteroaryl group(s), lower alkanoyl group, aroyl group, halogeno lower alkanoyl group, lower alkylsulfonyl group, arylsulfonyl group, heteroarylsulfonyl group, halogeno alkylsulfonyl group, lower alkyloxycarbonyl group, aryl-substituted lower alkyloxycarbonyl groups, substituted or unsubstituted carbamoyl group, and substituted or unsubstituted thiocarbamoyl group. Ammonium groups include a trialkylammonium group, for example.

Since phenylalanine compounds of the formula (1) of the present invention include asymmetric carbons, optical isomers are possible. The compounds of the present invention include such optical isomers and L-form thereof is preferable.

As for the compounds of which a diastereomer(s) exists, the diastereomer(s) and diastereomer mixture(s) are included in the compounds of the present invention. Further, since phenylalanine compounds of the formula (1) of the present invention include mobile hydrogen atoms, various tautomers are possible, and the compounds of the present invention include such tautomers. A carboxyl group in the compounds of the present invention may be substituted with a suitable substituent(s) that are converted into a carboxyl group *in vivo*. Examples of such substituents include a lower alkoxycarbonyl group.

When the compounds of the formula (1) of the present invention can form salts thereof, such salts should be pharmaceutically acceptable ones. For example, to acidic groups such as a carboxyl group in the formula, examples of the salts include salts with alkali metals (such as sodium, potassium, and ammonium); salts with alkaline earth metals (such as calcium and magnesium); aluminum salts; zinc salts; salts with organic amines (such as triethylamine, ethanolamine, morpholine, piperidine, and dicyclohexylamine); and salts with basic amino acids (such as arginine and lysine).

To basic groups in case that a basic group(s) exists in the formula, examples of the salts include salts with inorganic acids (such as hydrochloric acid, sulfuric acid, and phosphoric acid); salts with organic carboxylic acids (such as acetic acid, citric acid, benzoic acid, maleic acid, fumaric acid, tartaric acid, and succinic acid); and salts with organic sulfonic acids (such as methanesulfonic acid and p-toluenesulfonic acid). Regarding methods for forming salts, salts can be obtained by mixing the compounds of the formula (1) with a necessary acid or base at a suitable quantitative ratio in a solvent(s) or dispersant(s); or by conducting cation exchange or anion exchange to other form of salts.

The compounds of the present invention also include solvates such as hydrates and alcohol adducts of the compounds of the formula (1).

The compound (I) or pharmaceutically acceptable salts thereof can be produced by the method described in WO02-16329 (Patent Literature 1). Examples of the Compound (I) include Examples 1 to 213 described in WO02-16329 (Patent Literature 1). The description of WO02-16329 is included in the present specification.

The phenylalanine compound of the formula (1) is preferably a compound wherein R1 represents a methyl group or ethyl group; and R2, R3, and R4 represent a hydrogen atom, halogen atom, hydroxyl group, substituted low alkyl group, substituted lower alkenyl group, substituted lower alkynyl group, heteroaryl group, hydroxy lower alkyl group, amino group substituted with a lower alkyl group(s), or carbamoyl group substituted with a lower alkyl group(s), wherein substituents in the substituted low alkyl group, the substituted lower alkenyl group and the substituted lower alkynyl group include an amino group, amino group substituted with a lower alkyl group(s), carboxyl group, lower alkoxycarbonyl group, cyano group, lower alkylthio group, and lower alkylsulfonyl group.

The compound (I) or pharmaceutically acceptable salts thereof are preferably Examples 1, 108, 162, 169, 122, 66, 91, 99, 89, 75, 147, 148, 202, 201, 196, 193, 198 or 197 described in WO02-16329 (Patent Literature 1). They are shown as follows.

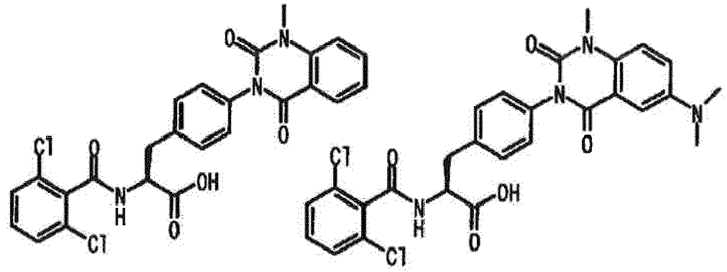

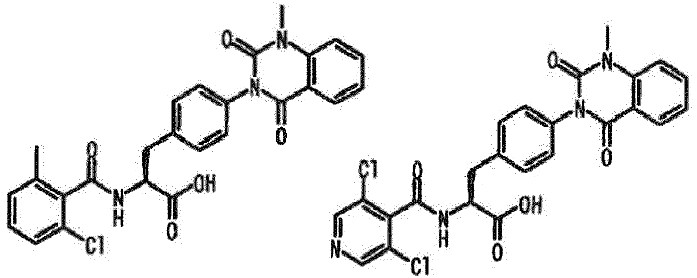

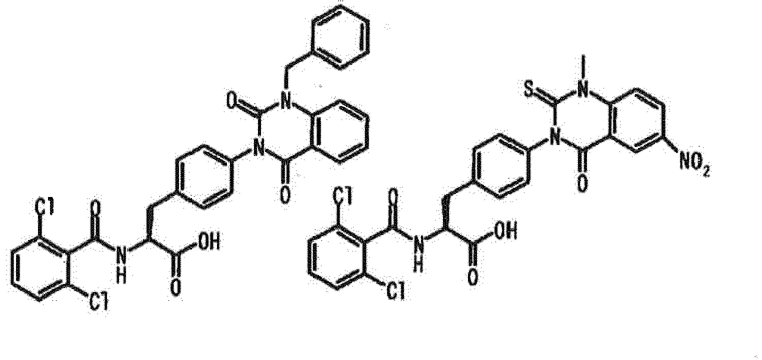

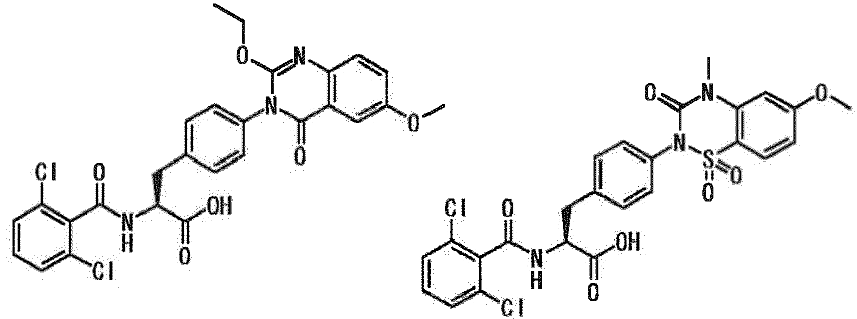

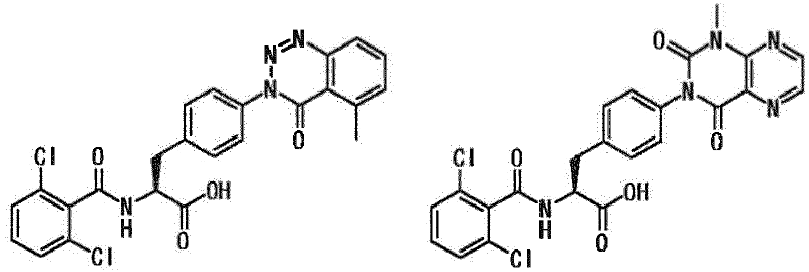

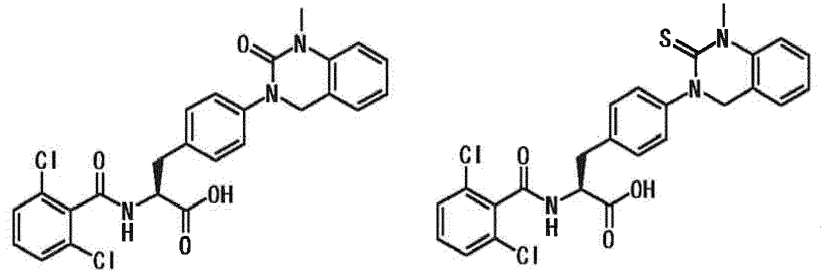

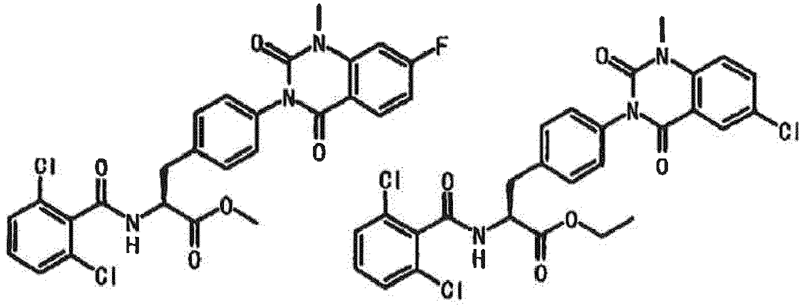

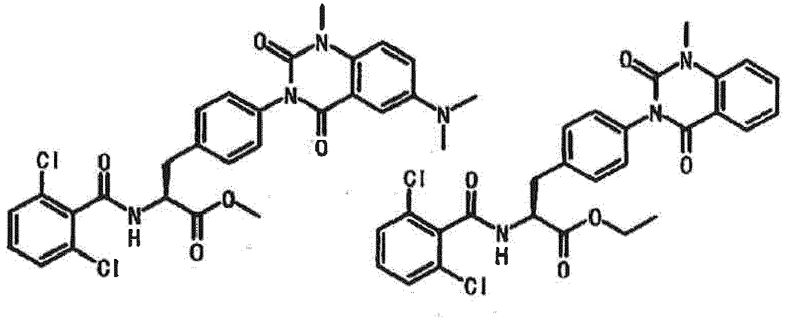

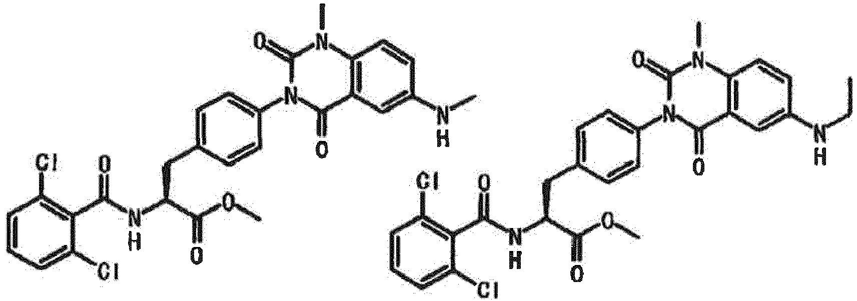

Most preferable one is Example 196 described in WO02-16329 (Patent Literature 1). The present compound (hereinafter referred to as a compound (A)) is shown as follows.

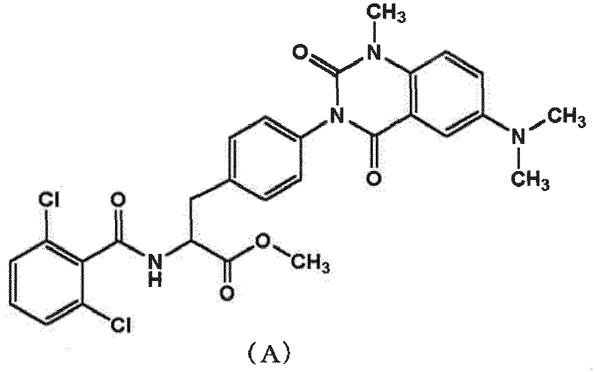

A "sustained-release oral administration preparation" of the present invention also includes "delayed release type oral administration preparations" in addition to so-called "sustained-release type oral administration preparations."

The above "sustained-release type oral administration preparations" are usually preparations which are planned to gradually release drugs from the preparations within digestive tracts regardless of in the stomach or the intestines. The present invention includes both a controlled release type, which maintains the constant concentration of drugs in blood plasma and continuance of the drugs' action is expected; and a prolonged release type, which cannot maintain the constant concentration of drugs in blood plasma, but continuance of the drugs' action can be expected.

Besides, the above "delayed release type oral administration preparations" are preparations which are planned to delay the transport time of preparations from the stomach to the small bowel or to release drugs from the preparations in the bowel parts rather than the stomach. The present invention includes enteric coated preparations.

Examples of sustained-release oral administration preparations of the present invention include sustained-release preparations which are prepared by sustained-release techniques such as a method with a controlled release membrane, a matrix method, a method with large granules, a capillary phenomenon method, a method for chemically lowering the speed of dissolution and a method for lowering the speed of dissolution by evaporation. Among them, methods with a controlled release membrane include coating, microcapsuling, film/tubing, a porous membrane method, a cyclodextrin method, and a liposome method. In preparations of the present invention, sustained-release preparations using the above sustained-release techniques are included, and the sustained-release preparations using a method with a controlled release membrane, a matrix method, and a method with large granules are preferable among them. Coating is preferable among methods with a controlled release membrane.

As for sustained-release preparations using coating, there is a coating preparation, for example, and as for sustained-release preparations using a matrix method, there is a matrix preparation, for example. The coating preparation and matrix preparation are preferable as sustained-release oral administration preparations in the present invention.

"Coating preparations" in the present invention are those of which core part containing the drugs (the compound (I) or the compound (A), or pharmaceutically acceptable salts thereof) is coated with a coating material(s) (a water-soluble coating material(s) or a water-insoluble coating material(s)).

In coating preparations using "a water-soluble coating material(s)," the drugs are exposed and released outside because the coating material(s) gradually dissolves in digestive tracts or decays. Other than that, the drugs are exposed and released outside because the coating material(s) dissolves in bowels or disintegrates when being transported to bowels, not in the stomach. The present preparations can control the release of drugs from the preparations by rates of the dissolution or disintegration of "a water-soluble coating material(s)." Preparations coated with a water-soluble coating material(s) include, for example, enteric coated tablets (such as tablets and pills), enteric granules, enteric fine granules, enteric capsules, and suspensions or emulsions containing drugs coated with enteric materials.

For example, in case of coating preparation treated with a water-soluble coating, the dissolution rate of the compound (1) in the preparation depends on pH and is preferably less than 20% to the dissolution rate for 1 hour in pH1.2 (The Japanese Pharmacopoeia Fourteenth Edition, No. 1 solution of the disintegration test).

A "water-soluble coating material(s)" is a pharmaceutically acceptable substance that dissolves in gastrointestinal solutions, and includes the following compounds.

### 1. Acrylic acid derivatives

methacrylic acid copolymer L, methacrylic acid copolymer S, methacrylic acid copolymer LD, aminoalkyl methacrylate copolymer E, and the like.

### 2. Cellulose derivatives

hydroxypropylmethylcellulose phthalate, hydroxypropylmethylcellulose acetate succinate, carboxymethylethylcellulose, cellulose acetate phthalate, hydroxyethylcellulose, hydroxypropylcellulose, hydroxypropylmethyl- cellulose, methylcellulose, methylhydroxyethylcellulose, opadry, carmellose calcium, carmellose sodium, and the like.

### 3. Vinyl derivatives

polyvinylacetal diethylaminoacetate, polyvinylpyrrolidone, polyvinyl alcohol, and the like.

### 4. Natural polymers and sugars

shellac, gelatin, agar, gum Arabic, pullulan, keratin, and the like.

One, two or more of these "water-soluble coating materials" can be used.

They are preferably methacrylic acid copolymer L, methacrylic acid copolymer S, methacrylic acid copolymer LD, hydroxypropylmethylcellulose phthalate, hydroxypropylmethylcellulose acetate succinate, carboxymethylethylcellulose, cellulose acetate phthalate, polyvinylacetal diethylaminoacetate, shellac, gelatin, and agar.

They are more particularly preferably methacrylic acid copolymer L, methacrylic acid copolymer S, methacrylic acid copolymer LD, hydroxypropylmethylcellulose phthalate, and hydroxypropylmethylcellulose acetate succinate.

On the other hand, in coating preparations using "a water-insoluble coating material(s)," the drugs are gradually released outside via the coating material(s). The present preparations can control the release of drugs by the interruption of the "water-insoluble coating material(s)." Preparations coated with a water-insoluble coating material(s) include, for example, sustained-release coated tablets (such as tablets and pills), sustained-release granules, sustained-release fine granules, sustained-release capsules, and suspensions or emulsions containing drugs coated with sustained-release materials.

For example, in case of coating preparation treated with a water-insoluble coating material(s), the dissolution rate of the compound (1) from the preparation 1 hour later is preferably less than 50% in pH6.8 (1/4 diluted McIlvaine buffer containing 5% (W/V) sodium dodecyl sulfate).

The above dissolution rate can be calculated by conducting the dissolution test in accordance with the Japanese Pharmacopoeia Fourteenth Edition No. 2 in conditions of: paddle rotation speed: 50rpm, test solution quantity: 900mL, test solution: 1/4 diluted McIlvaien buffer (pH6.8) + 5% (W/V) sodium dodecyl sulfate, test solution temperature: 37±0.5°C.

A "water-insoluble coating material(s)" is a pharmaceutically acceptable substance that hardly dissolves in gastrointestinal solutions, and includes the following compounds.

### 1. Cellulose derivatives

ethylcellulose, acetylcellulose, cellulose acetate, cellulose propionate, butyl cellulose, and the like.

### 2. Acrylic acid derivatives

aminoalkyl methacrylate copolymer RS, aminoalkyl methacrylate copolymer RL, ethyl acrylate methyl methacrylate copolymer/emulsion, and the like.

### 3. Vinyl derivatives

polyvinylacetate, polyvinylbutylate, and the like.

### 4. Esters

glyceride of a fatty acid of plant and animal origin and mixtures thereof, hydrogenated oils of glyceride of plant and animal origin (such as hydrogenated castor oil and hydrogenated canola oil), glyceride of fatty acids such as an oleic acid, a linoleic acid, a linolenic acid and a linosinic acid, and mixtures thereof, cholesteryl palmitate, palmitates of plant sterol, and the like.

### 5. Fatty acids

lauric acid, tridecanoic acid, myristic acid, pentadecanoic acid, palmitic acid, margaric acid, stearic acid, nanodecanoic acid, arachidic acid, behenic acid, lignoceric acid, cerotic acid, montanic acid, oleic acid, linoleic acid, linolenic acid, capric acid, caproic acid, and the like.

### 6. Alcohols

pentadecanol, hexadecanol, heptadecanol, octadecanol, nanodecanol, eicosanol, wool alcohols, cholesterol, and the like.

### 7. Surfactants

polyoxyethylene hydrogenated castor oil 10, 30, sucrose fatty acid ester, sorbitan tri-fatty acid ester, sorbitan sesqui-fatty acid ester, sorbitan mono-fatty acid ester, glyceryl mono-fatty acid ester, and the like.

One, two or more of these "water-insoluble coating materials" can be used.

They are preferably ethylcellulose, acetylcellulose, cellulose acetate, cellulose propionate, aminoalkyl methacrylate copolymer RS, aminoalkyl methacrylate copolymer RL, ethyl acrylate methyl methacrylate copolymer/emulsion, polyvinylacetate, polyvinylbutylate, glyceride of a fatty acid of plant and animal origin and mixtures thereof, hydrogenated oils of glyceride of plant and animal origin (such as hydrogenated castor oil and hydrogenated canola oil), and glyceride of fatty acids such as an oleic acid, linoleic acid, linolenic acid and linosinic acid, and mixtures thereof.

The coating quantity of water-soluble coating materials or water-insoluble coating materials is 1 to 50 wt% of the solid content coverage of the preparation in tablets, preferably 2 to 30 wt% thereof and further preferably 5 to 30 wt%; and 1 to 100 wt% thereof in granules, preferably 2 to 50 wt% thereof and further preferably 5 to 50 wt%. In this connection, the solid content coverage indicates the solid content quantity (part by weight) of a coating agent coating a tablet to 100 parts by weight of crude tablets before coating (same as below).

The coated preparations of the present invention may have, for example, "the compound (I) or the compound (A), or pharmaceutically acceptable salts thereof', or the mixture of "the compound (I) or the compound (A), or pharmaceutically acceptable salts thereof" and a pharmaceutically acceptable additive(s) as the quick release part in the coated core part. Particularly, the core part is preferably formed by the solid dispersions wherein the compound (I), the compound (A) or pharmaceutically acceptable salts thereof are dispersed in water-soluble polymeric substances in amorphous state.

Such solid dispersions can be prepared using the water-soluble polymeric substances by applying either steps of (i) dissolving or dispersing the above compound (I) or pharmaceutically acceptable salts thereof in an organic solvent(s) together with a water-soluble polymeric substance(s), and then removing the organic solvent(s); (ii) dissolving or dispersing the above compound (I) or pharmaceutically acceptable salts thereof in a water-soluble polymeric substance(s) under heating, and then cooling the mixture; (iii) dissolving or dispersing the above compound (I) or pharmaceutically acceptable salts thereof in a water-soluble polymeric substance(s) under heating and under pressure, and then cooling the mixture; and (iv) mixing the above compound (I) or pharmaceutically acceptable salts thereof together with a water-soluble polymeric substance(s), and then crushing the mixture.

The coating part of the coating preparations of the present invention may contain "the compound (I) or the compound (A), or pharmaceutically acceptable salts thereof' themselves.

The coating preparations of the present invention can be produced in accordance with the ordinary methods by spray-drying to the core part containing the drugs an organic solvent(s) such as ethanol or the water-soluble coating material(s) that is dissolved or dispersed in water, using fluid bed granulation coating equipment, centrifugal fluid coating equipment, or vented drying coating equipment.

The "matrix preparations" in the present invention are those wherein the drugs (the compound (I) or the compound (A), or pharmaceutically acceptable salts thereof) and water-soluble matrix materials are contained and the preparations having a matrix structure by mixture of the drugs and the water-soluble matrix materials.

In the matrix preparations using the "water-soluble matrix materials", the drugs are gradually released outside since the water-soluble matrix materials dissolve from the outside and the drugs also dissolve as they expose. The present preparations can control the release of drugs from the preparations by the dissolution or disintegration speed of the "water-soluble matrix materials". The matrix preparations wherein the water-soluble matrix materials are used include matrix tablets, spantab tablets (double release tablets), lontab tablets (dry-coated tablets) and triple release tablets (inner core tablets).

For example, in the matrix preparations, the dissolution rate of the compound (1) from the preparations 1 hour later is preferably less than 50% in pH6.8 (1/4 diluted McIlvaine buffer containing 5% (W/V) sodium dodecyl sulfate).

The above dissolution rate can be obtained in accordance with the Japanese Pharmacopoeia Fourteenth Edition No. 2 by conducting the dissolution test in the conditions of paddle rotation speed: 50rpm, test solution quantity: 900mL, test solution: 1/4 diluted McIlvaien buffer (pH6.8) + 5% (W/V) sodium dodecyl sulfate, and test solution temperature of 37±0.5°C.

The "water-soluble matrix materials" are the substances that dissolve in digestive solutions and are pharmaceutically acceptable. They include the following compounds.

### 1. Acrylic acid derivatives

methacrylic acid copolymer L, methacrylic acid copolymer S, methacrylic acid copolymer LD, aminoalkyl methacrylate copolymer E, and the like.

### 2. Cellulose derivatives

hydroxypropylmethylcellulose phthalate, hydroxypropylmethylcellulose acetate succinate, carboxymethylethylcellulose, cellulose acetate phthalate, hydroxyethylcellulose, hydroxypropylcellulose, hydroxypropylmethyl- cellulose, methylcellulose, methylhydroxyethylcellulose, opadry, carmellose calcium, carmellose sodium, and the like.

### 3. Vinyl derivatives

polyvinylacetal diethylaminoacetate, polyvinylpyrrolidone, polyvinyl alcohol, and the like.

### 4. Natural polymers and sugars

shellac, gelatin, agar, gum Arabic, pullulan, keratin, and the like.

One or two or more of these "water-soluble matrix materials" can be used.

They are preferably aminoalkyl methacrylate copolymer E, hydroxyethylcellulose, hydroxypropylcellulose, hydroxypropylmethyl- cellulose, methylcellulose, methylhydroxyethylcellulose, opadry, carmellose calcium, carmellose sodium, polyvinylacetal diethylaminoacetate, polyvinylpyrrolidone, polyvinyl alcohol, shellac, gelatin, agar, gum Arabic or pullulan.

They are further more preferably hydroxypropylmethylcellulose or methylcellulose.

The weight ratio of "the compound (I) or the compound (A), or pharmaceutically acceptable salts thereof" to water-soluble matrix materials is preferably 1:0.1 to 1:20, and more preferably 1:05 to 1:10.

The matrix preparations of the present invention may have, for example, "the compound (I) or the compound (A), or pharmaceutically acceptable salts thereof' themselves, or the mixture of "the compound (I) or the compound (A), or pharmaceutically acceptable salts thereof' and a pharmaceutically acceptable additive(s) as the quick release part. Particularly, the matrix preparations are preferably formed by the solid dispersions wherein the compound (I), the compound (A) or pharmaceutically acceptable salts thereof are dispersed in water-soluble polymeric substances in amorphous state. These preparations include, for example, (i) preparations wherein the solid dispersions containing the drugs are granulated once, and then made as tablets by mixing powder and matrix materials; or (ii) the preparations wherein the solution containing the water-soluble polymeric substances for forming the solid dispersions and the drugs is spray-dried to the matrix materials.

The solid dispersions can be prepared using the water-soluble polymeric substances by applying either steps of (i) dissolving or dispersing the above compound (I) or pharmaceutically acceptable salts thereof in an organic solvent(s) together with a water-soluble polymeric substance(s), and then removing the organic solvent(s); (ii) dissolving or dispersing the above compound (I) or pharmaceutically acceptable salts thereof in a water-soluble polymeric substance(s) under heating, and then cooling the mixture; (iii) dissolving or dispersing the above compound (I) or pharmaceutically acceptable salts thereof in a water-soluble polymeric substance(s) under heating and under pressure, and then cooling the mixture; and (iv) mixing the above compound (I) or pharmaceutically acceptable salts thereof together with a water-soluble polymeric substance(s), and then crushing the mixture.

The matrix preparations of the present invention may be further coated with the water-soluble coating materials or the water-insoluble coating materials. In such a case, the water-soluble coating materials or the water-insoluble coating materials may contain "the compound (I) or the compound (A), or pharmaceutically acceptable salts thereof' themselves.

The matrix preparations of the present invention can be produced in accordance with the ordinary methods by directly compressing the mixture containing the drug and the water-soluble matrix materials to form tablets, or by granulating the mixture containing the drug and the water-soluble matrix materials with a granulator by extruding to form granulated substance or further compressing the granule to form tablets.

When preparing the sustained-release oral administration preparations of the present invention, additives can be added, if necessary, such as excipients like sugars, e.g. lactose, sucrose, glucose, hydrogenated maltose, mannitol, sorbitol, xylitol and trehalose, starches and derivatives thereof, e.g. partially α starch, dextrin, pullulan, corn starch and potato starch, celluloses, e.g. crystalline cellulose, microcrystalline cellulose, crystalline cellulose / carmellose sodium and hydroxypropyl cellulose, magnesium aluminometasilicate, silicon dioxide, light anhydrous silicic acid, and amino acids; coloring agents; flavoring agents, e.g. sucrose, aspartame, mannitol, dextran, saccharin, menthol, citric acid, tartaric acid, malic acid, ascorbic acid, sweet hydrangea leaves, fennel, ethanol, fructose, xylitol, glycyrrhizinic acid, purified sucrose, L-glutamine and cyclodextrin; disintegrating agents, e.g. hydroxypropyl cellulose, low-substituted hydroxypropyl cellulose, croscarmellose sodium, α starch, methylcellulose, sodium alginate, sodium carboxymethyl starch, carmellose calcium, carmellose sodium, crystalline cellulose and crystalline cellulose / carmellose sodium; and surfactants, e.g. sodium lauryl sulfate, polysolvate 80, sucrose fatty acid ester, polyoxyl 40 stearate, polyoxyethylene 60 hydrogenated caster oil, sorbitan monostearate and sorbitan monopalmitate.

Particularly, when further coating the matrix preparations, it is common to combine plasticizers, e.g. polyethyleneglycol, sucrose fatty acid ester, glycerine fatty acid ester, propylene glycol, triethyl citrate, caster oil and triacetin; or lubricants, e.g. magnesium stearate, talc, titanic oxide and light anhydrous silicic acid.

The present preparations can be either dosage forms of tablets, granules, capsules, microcapsules, pills, dispersants, or subtle granules, and particularly preferably tablets, granules, and capsules.

When granulating the present preparations, they are preferably granulated by agitation granulation, fluid bed granulation, extruding granulation, and spray-drying with agitation granulators, fluid bed granulators, extruding granulators, and spray-dryers and the like.

The administration amount is determined by the intended treatment effect, treatment period, age, and body weight, and the dosage of "the compound (I) or the compound (A), or pharmaceutically acceptable salts thereof" is 1 µ g to 5 µ g by oral administration per a day for adults.

The present invention includes the coating preparations or matrix preparations that can release the compound (I) or pharmaceutically acceptable salts thereof in the lower part of the small bowel.

The oral administration preparations of the present invention have excellent sustained-release property and continuous effects thereof. They are useful since they can decrease number of dosing the drugs and, as a result, improve QOL (quality of life) or compliance of patients.

Examples will further illustrate the sustained-release oral administration preparations of the present invention. They only explain the present invention and do not particularly limit the invention. Comparative examples indicate solid dispersion preparations that are not sustained-released. Meanwhile, in the following description, the compound (A) is Example 196 in WO02/16329 (Patent Literature 1). The following diluting tablets represent the tablets that are prepared by using inactive excipients such as granulated lactose.

### (Comparative Example 1) Tablets (not sustained-released)

792.0g of methanol was put into 216.0g of methylcellulose (SM4, Shin-Etsu Chemical Co., Ltd.) to moisten a whole methylcellulose. 3169.0g of dichloromethane was added thereto, stirred and dissolved. Then, 144.0g of the compound (A) was further added thereto, stirred and dissolved. Thus prepared solution was used as a spray solution mentioned below. 56.0g of partially α starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Parteck M200, MERCK) were put into the container of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated with hydroxypropylmethylcellulose (TC-5R, Shin-Etsu Chemical Co., Ltd.) to obtain a solid dispersion preparation (not sustained-released).

### (Comparative Example 2) Granules (not sustained-released)

792.9g of methanol was put into 216.0g of methylcellulose (SM4, Shin-Etsu Chemical Co., Ltd.) to moisten a whole methylcellulose. 3207.5g of dichloromethane was added thereto, stirred and dissolved. Then, 144.0g of the compound (A) was further added thereto, stirred and dissolved. Thus prepared solution was used as a spray solution mentioned below. 337.5g of purified sucrose spheres (Nonpareil 103, Freund Corporation) was put into the bath of a fluid bed granulator (FLO-1, Freund Corporation), mixed and dried at intake temperature of 90°C. Then, 2730g of the spray solution was sprayed in the conditions of the spray air pressure of 0.15Mpa; and spray speed of 30g/min. to conduct the fluid bed granulation. After the completion of spraying, the mixture was dried by the fluid bed granulator. Further, the mixture was sifted to 500 µ m to 850 µ m to obtain granules.

### (Comparative Example 3) Tablets (Not sustained released)

825.0g of methanol was put into 225.1g of methylcellulose (SM4, Shin-Etsu Chemical Co., Ltd.) to moisten a whole methylcellulose. 3300g of dichloromethane was added thereto, stirred and dissolved. Then, 150.1g of the compound (A) was further added thereto, stirred and dissolved. Thus prepared solution was used as a spray solution mentioned below. 56.0g of partially α starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Mannit P, TOWA CHEMICAL INDUSTRY CO., LTD.) were put into the bath of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated with hydroxypropylmethylcellulose (TC-5R, Shin-Etsu Chemical Co., Ltd.) to obtain a solid dispersion preparation (not sustained released).

### (Example 1) A coating preparation wherein methacrylic acid copolymer L was used as a water-soluble coating material

3.5g of Macrogol 6000 (NOF Corporation) and 94.0g of purified water were added to a solution wherein 35.0g of methacrylic acid/ethyl acrylate copolymer (Eudragit L100-55, Higuchi Shokai Co., Ltd. (Rohm GmbH)) was dissolved in 350.1g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 150.0g of the above coating solution with a coating machine (Highcoater HCT-MINI, Freund Corporation) to prepare Eudragit L100-55 coating tablets (solid part coverage of 10%).

### (Example 2) A coating preparation wherein methacrylic acid copolymer S was used as a water-soluble coating material

3.5g of Macrogol 6000 (NOF Corporation) and 94.0g of purified water were added to a solution wherein 35.0g of methacrylic acid/methyl acrylate copolymer (Eudragit S100, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 295.7g of the above coating solution with a coating machine (Highcoater HCT-MINI, Freund Corporation) to prepare Eudragit S100 coating tablets (solid part coverage of 10%).

### (Example 3) A coating preparation wherein hydroxypropylmethylcellulose phthalate was used as a water-soluble coating material

3.5g of Macrogol 6000 (NOF Corporation) was added to a solution wherein 35.0g of hydroxypropylmethylcellulose phthalate (HP-55, Shin-Etsu Chemical Co., Ltd.) was dissolved in the mixed solution of 350.0g of ethanol and 91.5g of purified water, stirred and dissolved. Then, 5.0g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 332.4g of the above coating solution with a coating machine (Highcoater HCT-MINI, Freund Corporation) to prepare HP-55 coating tablets (solid part coverage of 10%).

### (Example 4) A coating preparation wherein ethylcellulose was used as a water-insoluble coating material

3.0g of triethyl citrate (Culter Food Science Co., Ltd.) and 75.0g of purified water were added to a solution wherein 30.0g of ethylcellulose (Ethocel STD-7P, Dow Chem.) was dissolved in 425.2g of ethanol, stirred and dissolved. Then, 15.0g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 295.0g of the above coating solution with a coating machine (Highcoater HCT-MINI, Freund Corporation) to prepare Etcell STD-7P coating tablets (solid part coverage of 10%).

### (Example 5) A coating preparation wherein methacrylic acid copolymer L was used as a water-soluble coating material

3.5g of Macrogol 6000 (NOF Corporation) and 94.0g of purified water were added to a solution wherein 35.0g of methacrylic acid/ethyl acrylate copolymer (Eudragit L100-55, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 60g of the uncoated tablets obtained in Comparative Example 1 and 240g of diluted tablets were film coated using 321.4g of the above coating solution with a coating machine (Highcoater HCT-MINI, Freund Corporation) to prepare Eudragit L100-55 coating tablets (solid part coverage of 15%).

### (Example 6) A coating preparation wherein methacrylic acid copolymer S was used as a water-soluble coating material

3.5g of Macrogol 6000 (NOF Corporation) and 94.0g of purified water were added to a solution wherein 35.0g of methacrylic acid/methyl acrylate copolymer (Eudragit S100, Higuchi Shokai Co., Ltd. (Rohm GmbH)) was dissolved in 350.0g of ethanol, stirred and dissolved. Then, 17.5g of talc (Matsumura Sangyo Co., Ltd.) was added thereto, stirred and dispersed to prepare a coating solution. 30g of the Eudragit S100 coating tablets (solid part coverage of 10%) obtained in Example 2 and 270g of diluted tablets were film coated using 85.0g of the above coating solution with a coating apparatus (Highcoater HCT-MINI, Freund Corporation) to prepare Eudragit S100 coating tablets (solid part coverage of 15%).

### (Example 7) A coating preparation wherein hydroxypropylmethyl- cellulose phthalate was used as a water-soluble coating material

3.5g of Macrogol 6000 (NOF Corporation) was added to a solution wherein 35.0g of hydroxypropylmethylcellulose phthalate (HP-55, Shin-Etsu Chemical Co., Ltd.) was dissolved in the mixed solution of 350.0g of ethanol and 91.5g of purified water, stirred and dissolved. Then, 5.0g of talc (Matsumura Sangyo Co., Ltd.) was added thereto, stirred and dispersed to prepare a coating solution. 30g of the HP-55 coating tablets (solid part coverage of 10%) obtained in Example 3 and 240g of diluted tablets were film coated using 104.9g of the above coating solution with a coating machine (Highcoater HCT-MINI, Freund Corporation) to prepare HP-55 coating tablets (solid part coverage of 15%).

### (Example 8) A coating preparation wherein ethylcellulose was used as a water-insoluble coating material

3.0g of triethyl citrate (Culter Food Science Co., Ltd.) and 75.0g of purified water were added to a solution wherein 30.0g of ethylcellulose (Ethocel STD-7P, Dow Chem.) was dissolved in 425.0g of ethanol, stirred and dissolved. Then, 17.5g of talc (Matsumura Sangyo Co., Ltd.) was added thereto, stirred and dispersed to prepare a coating solution. 30g of the Etocel STD-7P coating tablets (solid part coverage of 10%) obtained in Example 4 and 240g of diluted tablets were film coated using 140.0g of the above coating solution with a coating apparatus (Highcoater HCT-MINI, Freund Corporation) to prepare Etocel STD-7P coating tablets (solid part coverage of 15%).

### (Example 9) A matrix preparation wherein hydroxypropylmethyl- cellulose was used as a water-soluble matrix material

665.9g of methanol was put into 180.0g of methylcellulose (SM4, Shin-Etsu Chemical Co., Ltd.) to moisten a whole methylcellulose. 2640.7g of dichloromethane was added thereto, stirred and dissolved. Then, 120.1g of the compound (A) was further added thereto, stirred and dissolved. Thus prepared solution was used as a spray solution mentioned below. 44.8g of partially α starch (PCS PC-10, Asahi Kasei Corporation), 35.8g of croscarmellose sodium (Ac-Di-Sol, Asahi Kasei Corporation), 84.0g of crystalline cellulose (Avicel PH102, Asahi Kasei Corporation), 17.4g of mannitol (Parteck M200, MERCK) and 112.0g of hydroxypropylmethylcellulose 2906 (Metolose 65SH-4000, Shin-Etsu Chemical Co., Ltd.) were put into the container of a fluid bed granulator (FLO-1, Freund Corporation), mixed and dried at intake temperature of 90°C. Then, 3404.9g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated to obtain a matrix preparation with hydroxypropylmethylcellulose 2906 (Metolose 65SH-4000).

### (Example 10) A matrix preparation No. 2 wherein hydroxypropylmethylcellulose was used as a water-soluble matrix material

664.5g of methanol was put into 180.0g of methylcellulose (SM4, Shin-Etsu Chemical Co., Ltd.) to moisten a whole methylcellulose. 2645.7g of dichloromethane was added thereto, stirred and dissolved. Then, 120.0g of the compound (A) was further added thereto, stirred and dissolved. Thus prepared solution was used as a spray solution mentioned below. 44.8g of partially α starch (PCS PC-10, Asahi Kasei Corporation), 35.8g of croscarmellose sodium (Ac-Di-Sol, Asahi Kasei Corporation), 84.0g of crystalline cellulose (Avicel PH102, Asahi Kasei Corporation), 17.4g of mannitol (Mannit P, TOWA CHEMICAL INDUSTRY CO., LTD.) and 112.0g of hydroxypropylmethylcellulose 2208 (Metolose 90SH-4000SR, Shin-Etsu Chemical Co., Ltd.) were put into the container of a fluid bed granulator (FLO-1, Freund Corporation), mixed and dried at intake temperature of 90°C. Then, 3390.0g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain a matrix preparation with hydroxypropylmethylcellulose 2208 (Metolose 90SH-4000SR).

### (Example 11) A matrix preparation No. 3 wherein hydroxypropylmethylcellulose was used as a water-soluble matrix material (Two-folded amounts of hydroxypropylmethylcellulose)

660.1g of methanol was put into 180.0g of methylcellulose (SM4, Shin-Etsu Chemical Co., Ltd.) to moisten a whole methylcellulose. 2639.6g of dichloromethane was added thereto, stirred and dissolved. Then, 120.0g of the compound (A) was further added thereto, stirred and dissolved. Thus prepared solution was used as a spray solution mentioned below. 35.8g of partially α starch (PCS PC-10, Asahi Kasei Corporation), 28.7g of croscarmellose sodium (Ac-Di-Sol, Asahi Kasei Corporation), 53.8g of crystalline cellulose (Avicel PH102, Asahi Kasei Corporation), 13.4g of mannitol (Mannit P, TOWA CHEMICAL INDUSTRY CO., LTD.) and 179.2g of hydroxypropylmethylcellulose 2906 (Metolose 65SH-4000, Shin-Etsu Chemical Co., Ltd.) were put into the container of a fluid bed granulator (FLO-1, Freund Corporation), mixed and dried at intake temperature of 90°C. Then, 3390.0g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain a matrix preparation with hydroxypropylmethylcellulose 2906 (Metolose 65SH-4000).

### (Example 12) A matrix preparation No. 4 wherein hydroxypropylmethylcellulose was used as a water-soluble matrix material (Three-folded amounts of hydroxypropylmethylcellulose)

660.8g of methanol was put into 180.0g of methylcellulose (SM4, Shin-Etsu Chemical Co., Ltd.) to moisten a whole methylcellulose. 2643.3g of dichloromethane was added thereto, stirred and dissolved. Then, 120.0g of the compound (A) was further added thereto, stirred and dissolved. Thus prepared solution was used as a spray solution mentioned below. 35.8g of partially α starch (PCS PC-10, Asahi Kasei Corporation), 28.7g of croscarmellose sodium (Ac-Di-Sol, Asahi Kasei Corporation), 53.8g of crystalline cellulose (Avicel PH102, Asahi Kasei Corporation), 13.0g of mannitol (Mannit P, TOWA CHEMICAL INDUSTRY CO., LTD.) and 268.8g of hydroxypropylmethylcellulose 2906 (Metolose 65SH-4000, Shin-Etsu Chemical Co., Ltd.) were put into the container of a fluid bed granulator (FLO-1, Freund Corporation), mixed and dried at intake temperature of 90°C. Then, 3390.0g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate was added to the obtained granules and tableted to obtain uncoated tablets. The obtained uncoated tablets were film coated to obtain a matrix preparation with hydroxypropylmethylcellulose 2906 (Metolose 65SH-4000).

### (Example 13) A matrix preparation No. 5 wherein hydroxypropylmethylcellulose was used as a water-soluble matrix material (External addition of hydroxypropylmethylcellulose)

792.0g of methanol was put into 216.0g of methylcellulose (SM4, Shin-Etsu Chemical Co., Ltd.) to moisten a whole methylcellulose. 3169.8g of dichloromethane was added thereto, stirred and dissolved. Then, 144.0g of the compound (A) was further added thereto, stirred and dissolved. Thus prepared solution was used as a spray solution mentioned below. 56.0g of partially α starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Mannit P, TOWA CHEMICAL INDUSTRY CO., LTD.) were put into the container of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate and the equal amount and three-folded amount of hydroxypropylmethylcellulose 2208 (Metolose 90SH-4000SR) to the compound (A) were added to the obtained granules and tableted to obtain an externally added matrix preparation with hydroxypropylmethylcellulose 2208 (Metolose 90SH-4000SR).

### (Example 14) A matrix preparation wherein polyvinyl alcohol was used as a water-soluble matrix material

792.0g of methanol was put into 216.0g of methylcellulose (SM4, Shin-Etsu Chemical Co., Ltd.) to moisten a whole methylcellulose. 3169.8g of dichloromethane was added thereto, stirred and dissolved. Then, 144.0g of the compound (A) was further added thereto, stirred and dissolved. Thus prepared solution was used as a spray solution mentioned below. 56.0g of partially α starch (PCS PC-10, Asahi Kasei Corporation), 44.8g of croscarmellose sodium (Ac-Di-Sol, Asahi Kasei Corporation), 84.0g of crystalline cellulose (Avicel PH102, Asahi Kasei Corporation) and 22.4g of mannitol (Mannit P, TOWA CHEMICAL INDUSTRY CO., LTD.) were put into the container of a fluid bed granulator (FLO-1, Freund Corporation), mixed and dried at intake temperature of 90°C. Then, 4230g of the spray solution was sprayed in the conditions of the spray air pressure of 0.15Mpa; and spray speed of 30g/min. to conduct the fluid bed granulation. After the completion of spraying, the mixture was dried by the fluid bed granulator to obtain granules. 0.5% magnesium stearate and the equal amount and two-folded amount of polyvinyl alcohol (Gohsenol EG-25, The Nippon Synthetic Chemical Industry Co.,Ltd.) to the compound (A) were added to the obtained granules and tableted to obtain an externally added matrix preparation with polyvinyl alcohol (Gohsenol EG-25, The Nippon Synthetic Chemical Industry Co.,Ltd.).

### (Example 15) A coating granulated preparation wherein methacrylic acid copolymer L was used as a water-soluble coating material

21.0g of Macrogol 6000 (NOF Corporation) and 572.7g of purified water were added to a solution wherein 210.3g of methacrylic acid/ethyl acrylate copolymer (Eudragit L100-55, Higuchi Shokai Co., Ltd. (Rohm GmbH)) was dissolved in 2100.6g of ethanol, stirred and dissolved. Then, 105.0g of talc (Matsumura Sangyo Co., Ltd.) was added thereto, stirred and dispersed to prepare a coating solution. 120g of the spherical granules obtained in Comparative Example 2 was coated using 1287.1g of the above coating solution with a fluid bed granulator (FLO-1, Freund Corporation) to prepare Eudragit L100-55 coating granules (solid part coverage of 61%).

### (Example 16) A capsule preparation with a hydroxypropylmethylcellulose acetate succinate capsule

93.2mg of the following granules A, 46.6mg of sodium hydrogen carbonate (Top-grade, Wako Pure Chemical Industries, Ltd.) and 46.6mg of L-tartaric acid were put into hydroxypropylmethylcellulose acetate succinate capsules (Shin-Etsu Chemical Co., Ltd.) and sealed with hydroxypropylmethylcellulose acetate succinate (Shin-Etsu Chemical Co., Ltd.) dissolved in acetone to prepare capsules.

The following granules A were produced as follows.

252kg of methylene chloride and 63kg of methanol were put into a tank, 30kg of methylcellulose (SM4, Shin-Etsu Chemical Co., Ltd.) was dissolved therein. Then, 10kg of croscarmellose sodium (Ac-Di-Sol, Asahi Kasei Corporation) was put therein. 252kg of methylene chloride and 63kg of methanol were put into another tank, 30kg of the compound (A) was dissolved therein, and mixed with the above dispersion solution.

All amount of the mixture was sprayed by a spray-dryer (Fuji Chemical Industry Co., Ltd.) in the conditions of heat input temperature of about 100°C, reed rate of 40kg/hr, and nozzle pressure of 0.15Mpa, and dried at 60°C for 20 hours. The obtained granules were mixed for 5 minutes by a nauta mixer (rotation: 50rpm, revolution: 2rpm) to obtain 62.71kg of solid dispersion granules. The obtained granules were compacted and crushed by a speed mill and Comil to obtain granules (A).

Test examples will further illustrate effect of sustained-release preparations of the present invention.

### (Test Example 1) A coating preparation

The dissolution test was conducted on the coating preparations of the present invention obtained in Examples 1 to 3 and 5 to 7 and the preparation obtained in Comparative Example 1. In the dissolution test, the acid resistance of 40mg of the compound (A) was evaluated from the dissolution rate two hours later in accordance with the Japanese Pharmacopoeia Fourteenth Edition No. 2 in conditions of: paddle rotation speed: 50rpm, test solution: solution 1.2 of USP (the United States Pharmacopoeia) 24, test solution temperature: 37±0.5°C.

### (Test Example 2) A coating preparation

The dissolution test was conducted on the coating preparations of the present invention obtained in Examples 1 to 4 and the preparation obtained in Comparative Example 1. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% (W/V) sodium dodecyl sulfate, test solution temperature: 37±0.5°C.

Similarly, the dissolution test was conducted on the coating preparations of the present invention obtained in Examples 5 to 8 and the preparation obtained in Comparative Example 1.

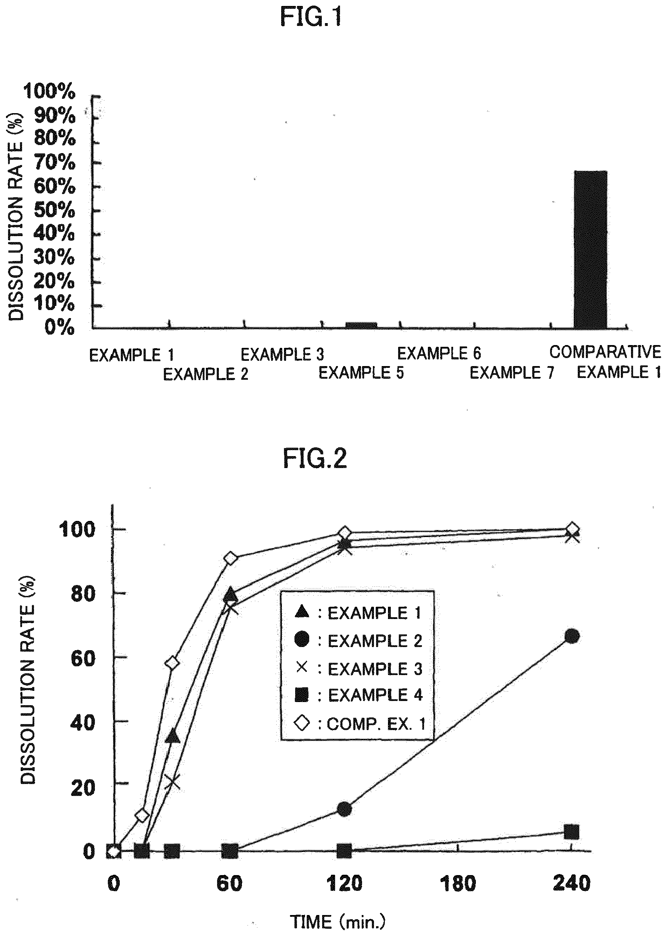

As indicated in Figs. 1 to 3, the release of the compound (A) from the preparation of Comparative Example 1 was rapid, while the release of the compound (A) from the preparations of the present invention obtained in Examples 1 to 4 and 5 to 8 was gradual. Particularly, the acid resistance was seen in the preparations of the present invention using a water-soluble coating material, obtained in Examples 1 to 3 and 5 to 7.

Thus, it has been clarified that the release of the compound (A) from the coating preparations of the present invention has been sufficiently controlled.

### (Test Example 3) A matrix preparation

The dissolution test was conducted on the matrix preparation of the present invention obtained in Example 9 and the preparation obtained in Comparative Example 1. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% SDS, test solution temperature: 37±0.5°C.

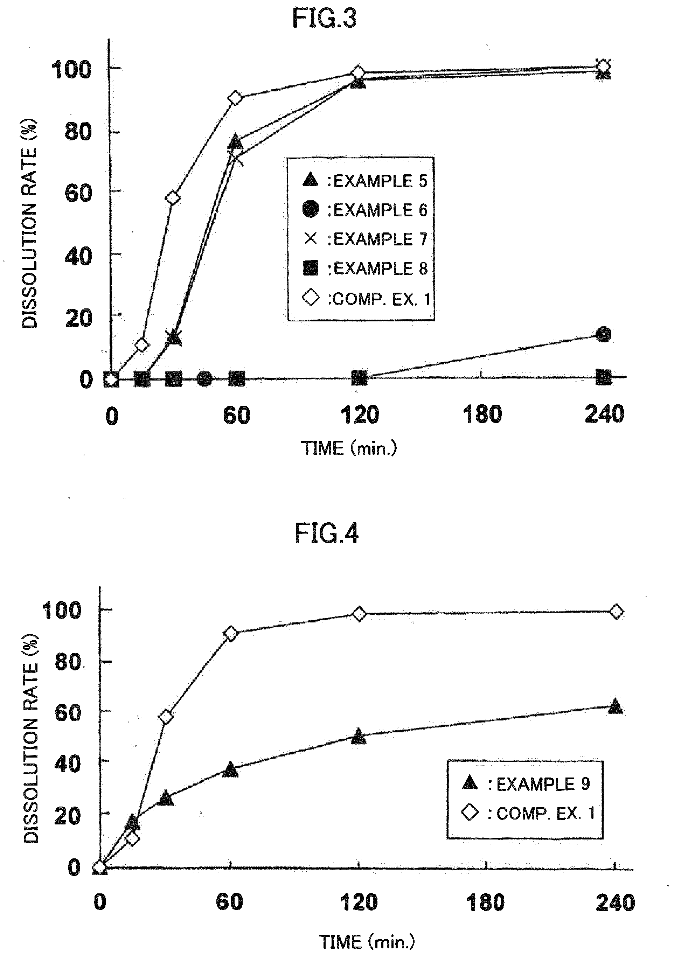

As indicated in Fig. 4, the release of the compound (A) from the preparation of Comparative Example 1 was rapid, while the release of the compound (A) from the preparation of the present invention obtained in Example 9 was gradual. Thus, it has been clarified that the release of the compound (A) from the matrix preparation of the present invention has been sufficiently controlled.

### (Test Example 4) Matrix dissolution test

The dissolution test was conducted on the matrix preparations of the present invention obtained in Examples 9 to 13 and the preparation obtained in Comparative Example 1. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% SDS, test solution temperature: 37±0.5°C.

### (Test Example 5) Acid resistance test

The dissolution test was conducted on the coating preparations of the present invention obtained in Examples 15 and 16 and the preparation obtained in Comparative Example 2. In the dissolution test, the acid resistance of 40mg of the compound (A) was evaluated from the dissolution rate two hours later in accordance with the Japanese Pharmacopoeia Fourteenth Edition No. 2 in conditions of: paddle rotation speed: 50rpm, test solution: JP 1.2 solution, test solution temperature: 37±0.5°C.

### (Test Example 6) Externally added matrix dissolution test (PVA)

The dissolution test was conducted on the matrix preparation of the present invention obtained in Example 14 and the preparation obtained in Comparative Example 1. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% SDS, test solution temperature: 37±0.5°C.

### (Test Example 7) Pharmacokinetic parameters (Tmax)

40mg equivalents of the preparations of the present invention obtained Examples 3, 15 and 16 and Comparative Examples 1 to 3 were orally administered to male beagle dogs under fasting. The samples of the blood plasma were taken before the administration and 0.25, 0.5, 1, 2, 4, 6, 8, 12 and 24 hours after starting the administration and the maximum blood concentrations (Tmax) were shown in Table 1 as pharmacokinetic parameters. In this connection, Tmax indicates the time to reach the maximum blood concentration.

**Table 1 Pharmacokinetic parameters (Tmax)**

| | Tmax(hr) |
|---|---|
| Comparative Exam. 2 | 0.5 |
| Comparative Exam. 3 | 1.3 |
| Example 3 | 3.7 |
| Example 15 | 1.2 |
| Example 16 | 4.0 |

### (Test Example 8) The dissolution test at pH6.8

The dissolution test was conducted on the preparations of the present invention (enteric coated preparations) obtained in Examples 15 and 16. The dissolution test was conducted to 40mg of the compound (A) in accordance with the Japanese Pharmacopoeia Fourteenth Edition No. 2 in conditions of: paddle rotation speed: 50rpm, test solution: 1/4 diluted McIlvaine buffer (pH6.8) + 5% SDS, test solution temperature: 37±0.5°C. The good release of the drugs was seen at pH6.8.

The present invention provides sustained-release oral administration preparations of "the compound (I) or the compound (A), or pharmaceutically acceptable salts thereof". The sustained-release oral administration preparations of the present invention have an α 4 integrin inhibiting activity and are useful as therapeutic agents or preventive agents for inflammatory diseases in which α 4 integrin-depending adhesion process participates in the pathology, rheumatoid arthritis, inflammatory bowel diseases, systemic erythematodes, multiple sclerosis, Sjogren's syndrome, asthma, psoriasis, allergy, diabetes, cardiovascular diseases, arterial sclerosis, restenosis, tumor proliferation, tumor metastasis and transplantation rejection.

Aspects of the present invention include the following:
[1] A sustained-release oral administration preparation containing a phenylalanine compound of the following formula (1) or pharmaceutically acceptable salts thereof as an active ingredient: 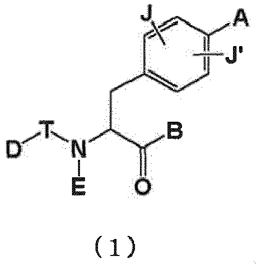 wherein A represents either of a following formula (2), (3), (3-1), or (3-2): 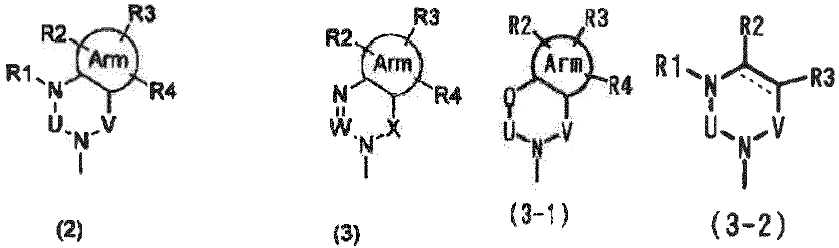 wherein Arm is a cyclic alkyl group or an aromatic ring having 0, 1, 2, 3, or 4 hetero atoms selected from the group consisting of an oxygen atom, sulfur atom and nitrogen atom; a combined line of a solid line and a dotted line in the formula (3-2) represents a single bond or a double bond; U, V, and X represent C(=O), S(=O)₂, C(-R5)(-R6), C(=C(R5)(R6)), C(=S), S(=O), P(=O)(-OH), or P(-H)(=O); W represents C(-R7) or a nitrogen atom;
   wherein R1, R2, R3, R4, R5, R6 and R7 may be same or different from each other and represent a hydrogen atom, halogen atom, hydroxyl group, lower alkyl group, substituted low alkyl group, lower alkenyl group, substituted lower alkenyl group, lower alkynyl group, substituted lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), lower alkyl group substituted with a heteroaryl group(s), lower alkoxy group, lower alkylthio group, lower alkoxy group and lower alkylthio group substituted with a cycloalkyl group(s) which may have a hetero atom(s) in the ring, lower alkoxy group and lower alkylthio group substituted with an aryl group(s), lower alkoxy group and lower alkylthio group substituted with a heteroaryl group(s), cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkylthio group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylsulfornyl group, substituted or unsubstituted sulfamoyl group, or ammonium group; R5 and R6 may bond together to form a ring, and in the ring, one or two oxygen atoms, nitrogen atoms or sulfur atoms may be included if necessary;
   B represents a hydroxyl group, lower alkoxy group, or hydroxylamino group;
   E represents a hydrogen atom, lower alkyl group, lower alkenyl group, lower alkynyl group, lower alkyl group substituted with a cycloalkyl group(s) which may have a hetero atom(s) in the ring, lower alkyl group substituted with an aryl group(s), or lower alkyl group substituted with a heteroaryl group(s);
   D represents a lower alkyl group, lower alkenyl group, lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), or lower alkyl group substituted with a heteroaryl group(s), lower alkoxy group, lower alkoxy group substituted with a cycloalkyl group(s) which may have a hetero atom(s) in the ring, lower alkoxy group substituted with an aryl group(s), lower alkoxy group substituted with a heteroaryl group(s), a cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylthio group, lower alkylsulfornyl group, or substituted or unsubstituted sulfamoyl group;
   E and D may bond together to form a ring, and in the ring, one or two oxygen atoms, nitrogen atoms or sulfur atoms may be included if necessary;
   T represents an interatomic bond, C(=O), C(=S), S(=O), S(=O)₂, N(H)-C(=O), or N(H)-C(=S); and
   J and J' may be same or different from each other and represents a hydrogen atom, halogen atom, lower alkyl group, lower alkyloxy group, or nitro group.
[2] The sustained-release oral administration preparation containing the compound of the formula (1) according to the above mentioned [1] or pharmaceutically acceptable salts thereof as an active ingredient in either form of a coating preparation or a matrix preparation.
[3] The sustained-release oral administration preparation according to the above mentioned [2], wherein the sustained-release oral administration preparation is the coating preparation.
[4] The sustained-release oral administration preparation according to the above mentioned [3], wherein the coating preparation is the preparation that the core part containing the active ingredient according to the above mentioned [2] is coated with a water-soluble coating material.
[5] The sustained-release oral administration preparation according to the above mentioned [4], wherein the water-soluble coating material is coated with at least one of acrylic acid derivatives, cellulose derivatives, vinyl derivatives, and natural polymers and sugars.
[6] The sustained-release oral administration preparation according to the above mentioned [4], wherein the water-soluble coating material is coated with at least one of methacrylic acid copolymer L, methacrylic acid copolymer S, methacrylic acid copolymer LD, hydroxypropylmethylcellulose phthalate, hydroxypropylmethylcellulose acetate succinate, carboxymethylethylcellulose, cellulose acetate phthalate, polyvinylacetal diethylaminoacetate, shellac, gelatin, and agar.
[7] The sustained-release oral administration preparation according to the above mentioned [4], wherein the water-soluble coating material is coated with at least one of methacrylic acid copolymer L, methacrylic acid copolymer S, methacrylic acid copolymer LD, hydroxypropylmethylcellulose phthalate, and hydroxypropylmethylcellulose acetate succinate.
[8] The sustained-release oral administration preparation according to any one of the above mentioned [4] to [7], wherein the solid content coverage is 1 to 50 wt% in tablets, and 1 to 100 wt% in granules.
[9] The sustained-release oral administration preparation according to any one of the above mentioned [4] to [7], wherein the solid content coverage is 2 to 30 wt% in tablets, and 2 to 50 wt% in granules.
[10] The sustained-release oral administration preparation according to the above mentioned [3], wherein the coating preparation is the preparation that the core part containing the active ingredient according to the above mentioned [2] is coated with a water-insoluble coating material.
[11] The sustained-release oral administration preparation according to the above mentioned [10], wherein the water-insoluble coating material is coated with at least one of cellulose derivatives, acrylic acid derivatives, vinyl derivatives, esters, fatty acids, alcohols and surfactants.
[12] The sustained-release oral administration preparation according to the above mentioned [10], wherein the water-insoluble coating material is coated with at least one of ethylcellulose, acetylcellulose, cellulose acetate, cellulose propionate, aminoalkyl methacrylate copolymer RS, aminoalkyl methacrylate copolymer RL, ethyl acrylate methyl methacrylate copolymer/emulsion, polyvinylacetate, polyvinylbutylate, glyceride of a fatty acid of plant and animal origin and mixtures thereof, hydrogenated oils of glyceride of plant and animal origin, and glyceride of fatty acids such as an oleic acid, linoleic acid, linolenic acid and linosinic acid, and mixtures thereof.
[13] The sustained-release oral administration preparation according to any one of the above mentioned [10] to [12], wherein the solid content coverage is 1 to 50 wt% in tablets, and 1 to 100 wt% in granules.
[14] The sustained-release oral administration preparation according to any one of the above mentioned [10] to [12], wherein the solid content coverage is 2 to 30 wt% in tablets, and 2 to 50 wt% in granules.
[15] The sustained-release oral administration preparation according to the above mentioned [2], wherein the sustained-release oral administration preparation is the matrix preparation.
[16] The sustained-release oral administration preparation according to the above mentioned [15], wherein the matrix preparation is the preparation containing the active ingredient according to above mentioned 2 and a water-soluble matrix material.
[17] The sustained-release oral administration preparation according to the above mentioned [16], wherein the water-soluble matrix material is at least one of acrylic acid derivatives, cellulose derivatives, vinyl derivatives, and natural polymers and sugars.
[18] The sustained-release oral administration preparation according to the above mentioned [16], wherein the water-soluble matrix material is at least one of aminoalkyl methacrylate copolymer E, hydroxyethylcellulose, hydroxypropylcellulose, hydroxypropylmethylcellulose, methylcellulose, methylhydroxyethylcellulose, opadry, carmellose calcium, carmellose sodium, polyvinylacetal diethylaminoacetate, polyvinylpyrrolidone, polyvinyl alcohol, shellac, gelatin, agar, gum Arabic and pullulan.
[19] The sustained-release oral administration preparation according to the above mentioned [16], wherein the water-soluble matrix material is at least one of hydroxypropylmethylcellulose and methylcellulose.
[20] The sustained-release oral administration preparation according to any one of the above mentioned [16] to [19], wherein the weight ratio of the active ingredient of the above mentioned [2] to the water-soluble matrix material is 1:0.1 to 1:20.
[21] The sustained-release oral administration preparation according to any one of the above mentioned [16] to [19], wherein the weight ratio of the active ingredient of the above mentioned [2] to the water-soluble matrix material is 1:0.5 to 1:10.
[22] The sustained-release oral administration preparation according to any one of the above mentioned [1] to [21], wherein the phenylalanine compound of the formula (1) or pharmaceutically acceptable salts thereof is a compound or pharmaceutically acceptable salts thereof wherein, in the formula (1), R1 represents a methyl group or ethyl group; and R2, R3, and R4 represent a hydrogen atom, halogen atom, hydroxyl group, substituted low alkyl group, substituted lower alkenyl group, substituted lower alkynyl group, heteroaryl group, hydroxy lower alkyl group, amino group substituted with a lower alkyl group(s), or carbamoyl group substituted with a lower alkyl group(s), wherein substituents in the substituted low alkyl group, the substituted lower alkenyl group and the substituted lower alkynyl group include an amino group, amino group substituted with a lower alkyl group(s), carboxyl group, lower alkoxycarbonyl group, cyano group, lower alkylthio group, and lower alkylsulfonyl group.
[23] The sustained-release oral administration preparation according to any one of the above mentioned [1] to [21], wherein the phenylalanine compound of the formula (1) or pharmaceutically acceptable salts thereof is a compound (A) or pharmaceutically acceptable salts thereof.

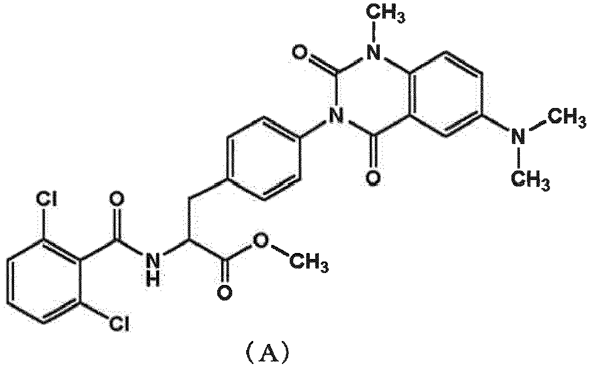

## Claims

1. An oral administration preparation containing a phenylalanine compound of the following formula (1) or pharmaceutically acceptable salts thereof as an active ingredient in the form of a matrix preparation: 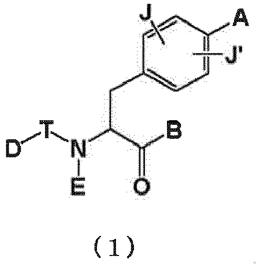 wherein A represents the following formula (2): 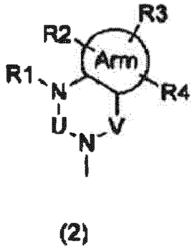 wherein Arm is a cyclic alkyl group or an aromatic ring having 0, 1, 2, 3, or 4 hetero atoms selected from the group consisting of an oxygen atom, sulfur atom and nitrogen atom; U and V represent C(=O), S(=O)₂, C(-R5)(-R6), C(=C(R5)(R6)), C(=S), S(=O), P(=O)(-OH), or P(-H)(=O);
wherein R1, R2, R3, R4, R5, and R6 may be same or different from each other and represent a hydrogen atom, halogen atom, hydroxyl group, lower alkyl group, substituted low alkyl group, lower alkenyl group, substituted lower alkenyl group, lower alkynyl group, substituted lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), lower alkyl group substituted with a heteroaryl group(s), lower alkoxy group, lower alkylthio group, lower alkoxy group and lower alkylthio group substituted with a cycloalkyl group(s) which may have a hetero atom(s) in the ring, lower alkoxy group and lower alkylthio group substituted with an aryl group(s), lower alkoxy group and lower alkylthio group substituted with a heteroaryl group(s), cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkylthio group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylsulfornyl group, substituted or unsubstituted sulfamoyl group, or ammonium group; R5 and R6 may bond together to form a ring, and in the ring, one or two oxygen atoms, nitrogen atoms or sulfur atoms may be included if necessary;
B represents a hydroxyl group, lower alkoxy group, or hydroxylamino group;
E represents a hydrogen atom, lower alkyl group, lower alkenyl group, lower alkynyl group, lower alkyl group substituted with a cycloalkyl group(s) which may have a hetero atom(s) in the ring, lower alkyl group substituted with an aryl group(s), or lower alkyl group substituted with a heteroaryl group(s);
D represents a lower alkyl group, lower alkenyl group, lower alkynyl group, cycloalkyl group which may have a hetero atom(s) in the ring, aryl group, heteroaryl group, lower alkyl group substituted with a cycloalkyl group(s) which may have a hetero atom(s) in the ring, lower alkyl group substituted with an aryl group(s), or lower alkyl group substituted with a heteroaryl group(s), lower alkoxy group, lower alkoxy group substituted with a cycloalkyl group(s) which may have a hetero atom(s) in the ring, lower alkoxy group substituted with an aryl group(s), lower alkoxy group substituted with a heteroaryl group(s), a cycloalkyloxy group which may have a hetero atom(s) in the ring, aryloxy group, heteroaryloxy group, hydroxy lower alkyl group, hydroxy lower alkenyl group, hydroxy lower alkoxy group, halogeno lower alkyl group, halogeno lower alkoxy group, halogeno lower alkenyl group, nitro group, cyano group, substituted or unsubstituted amino group, carboxyl group, lower alkyloxy carbonyl group, substituted or unsubstituted carbamoyl group, lower alkanoyl group, aroyl group, lower alkylthio group, lower alkylsulfornyl group, or substituted or unsubstituted sulfamoyl group;
E and D may bond together to form a ring, and in the ring, one or two oxygen atoms, nitrogen atoms or sulfur atoms may be included if necessary;
T represents an interatomic bond, C(=O), C(=S), S(=O), S(=O)₂, N(H)-C(=O), or N(H)-C(=S); and
J and J' may be same or different from each other and represents a hydrogen atom, halogen atom, lower alkyl group, lower alkyloxy group, or nitro group.

2. The oral administration preparation according to claim 1, wherein the matrix preparation is the preparation containing the active ingredient according to claim 1 and a water-soluble matrix material.

3. The oral administration preparation according to claim 2, wherein the water-soluble matrix material is at least one of acrylic acid derivatives, cellulose derivatives, vinyl derivatives, and natural polymers and sugars.

4. The oral administration preparation according to claim 2, wherein the water-soluble matrix material is at least one of aminoalkyl methacrylate copolymer E, hydroxyethylcellulose, hydroxypropylcellulose, hydroxypropylmethylcellulose, methylcellulose, methylhydroxyethylcellulose, opadry, carmellose calcium, carmellose sodium, polyvinylacetal diethylaminoacetate, polyvinylpyrrolidone, polyvinyl alcohol, shellac, gelatin, agar, gum Arabic and pullulan.

5. The oral administration preparation according to claim 2, wherein the water-soluble matrix material is at least one of hydroxypropylmethylcellulose and methylcellulose.

6. The oral administration preparation according to any one of claims 2 to 5, wherein the weight ratio of the active ingredient of claim 1 to the water-soluble matrix material is 1:0.1 to 1:20.

7. The oral administration preparation according to any one of claims 2 to 5, wherein the weight ratio of the active ingredient of claim 2 to the water-soluble matrix material is 1:0.5 to 1:10.

8. The oral administration preparation according to any one of claims 1 to 7, wherein the phenylalanine compound of the formula (1) or pharmaceutically acceptable salts thereof is a compound or pharmaceutically acceptable salts thereof wherein, in the formula (1), R1 represents a methyl group or ethyl group; and R2, R3, and R4 represent a hydrogen atom, halogen atom, hydroxyl group, substituted low alkyl group, substituted lower alkenyl group, substituted lower alkynyl group, heteroaryl group, hydroxy lower alkyl group, amino group substituted with a lower alkyl group(s), or carbamoyl group substituted with a lower alkyl group(s), wherein substituents in the substituted low alkyl group, the substituted lower alkenyl group and the substituted lower alkynyl group include an amino group, amino group substituted with a lower alkyl group(s), carboxyl group, lower alkoxycarbonyl group, cyano group, lower alkylthio group, and lower alkylsulfonyl group.

9. The oral administration preparation according to any one of claims 1 to 8, wherein the phenylalanine compound of the formula (1) or pharmaceutically acceptable salts thereof is a compound (A) or pharmaceutically acceptable salts thereof.

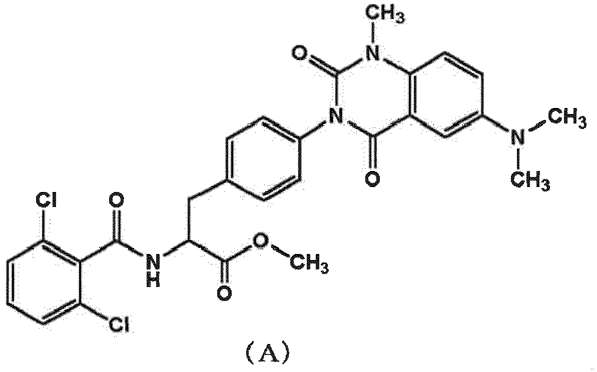

10. The oral administration preparation according to any one of the preceding claims which is a sustained release oral administration preparation.
